# FASCICULE DE BREVET EUROPEEN

(11) **EP 2 707 039 B1**
(45) Date de publication et mention de la délivrance du brevet: **01.03.2017**
(21) Numéro de dépôt: 12728650.8
(22) Date de dépôt: 10.05.2012
(51) Int. Cl.: F24F 3/14, A61L 9/015, A61L 2/20, A61L 9/03, F24F 3/16

(54) **DISPOSITIF DE DÉCONTAMINATION AÉRIENNE**
LUFTDEKONTAMINATIONSVORRICHTUNG
AIR DECONTAMINATION DEVICE

(30) Priorité: 11.05.2011 FR 1154059
(43) Date de publication de la demande: 19.03.2014
(73) Titulaire: Henriot, Philippe, 38138 Les Côtes d'Arey (FR)
(72) Inventeur: Henriot, Philippe, 38138 Les Côtes d'Arey (FR)
(74) Mandataire: Delorme, Nicolas
(86) Numéro de dépôt international: PCT/FR2012/051037
(87) Numéro de publication internationale: WO 2012/153068

(56) Documents cités:
- EP-A1- 0 774 263
- WO-A1-2004/020919
- WO-A1-2007/008205
- US-A1- 2005 084 415
- US-A1- 2008 135 635

## Description

La présente invention concerne un dispositif de décontamination aérienne.

La décontamination aérienne consiste à diffuser dans une atmosphère un produit ayant des propriétés microbicides en vue de réduire sensiblement le nombre de microbes vivant dans cette atmosphère. La décontamination aérienne permet subsidiairement de réduire le nombre de microbes présents sur les surfaces des objets au contact de cette atmosphère.

Il est connu de décontaminer des locaux grâce à des dispositifs de décontamination aérienne diffusant un produit microbicide, par exemple du glutaraldéhyde, de l'acide peracétique ou du peroxyde d'hydrogène. Pour ce dernier, de plus en plus prisé du fait de ses avantages, il existe deux modes de diffusion : la brumisation (diffusion du produit dilué à environ 3% sous forme de brouillard) et la vaporisation (diffusion du produit concentré à environ 35% sous forme de gaz). Ce dernier procédé est plus efficace du fait de la plus grande concentration, de la grande facilité de diffusion du gaz, de l'absence de dépôt de produit dans les conditions normales, et d'autres avantages. Par contre le produit concentré est très corrosif, alors que le produit dilué l'est très peu.

Cette diffusion gazeuse peut avoir lieu en circuit ouvert ou en circuit fermé. Par rapport à une diffusion en circuit ouvert, la diffusion en circuit fermé présente l'avantage de prévenir les fuites du produit microbicide hors de l'enceinte à décontaminer (en raison de sa toxicité pour l'homme) ou de prévenir sa dilution par un débit d'air extérieur entrant dans l'enceinte à décontaminer ce qui diminuerait son efficacité. La diffusion en circuit fermé offre en outre l'avantage de s'affranchir (du fait de la toxicité) de la nécessité de prévoir un réseau d'évacuation vers l'extérieur des locaux abritant l'enceinte, comme c'est le cas pour une diffusion dans un circuit ouvert.

Cependant, la vaporisation en circuit fermé d'un produit microbicide comme un liquide contenant du peroxyde d'hydrogène va conduire à une augmentation de l'humidité relative à l'intérieur de l'enceinte. En effet, le liquide contenant du peroxyde d'hydrogène diffusé dans l'enceinte présente une certaine teneur en eau d'une part (par exemple 65%), et la transformation du peroxyde d'hydrogène conduit à la formation d'eau d'autre part. L'augmentation de l'humidité relative peut conduire à une condensation du peroxyde d'hydrogène vaporisé. Les dépôts liquides de peroxyde d'hydrogène ainsi formés sont très corrosifs, alors que la forme gazeuse l'est beaucoup moins. Cela peut entraîner la corrosion des surfaces sensibles des objets présents dans l'enceinte sur lesquelles se sont formés les dépôts.

En outre, les dispositifs de décontamination existants sont généralement coûteux à l'achat et à l'exploitation, nécessitent parfois des opérations de maintenance fréquentes, et peuvent présenter un encombrement important ou une maniabilité faible.

Il est connu du document de brevet EP0774263 un appareil pour la stérilisation par la vapeur de peroxyde d'hydrogène. Toutefois, la circulation de l'air à l'intérieur de l'appareil décrit dans le document de brevet EP0774263 est assurée uniquement par un ventilateur qui ne permet pas de refouler l'air en aval à une pression sensiblement supérieure à celle de l'air en amont. L'humidité contenue dans l'air circulant dans l'appareil selon le document de brevet EP0177263 est donc séparée à pression atmosphérique. De plus, cette séparation est assurée par deux groupes frigorifiques fonctionnant en alternance et assurant la séparation par formation de glace sur leurs évaporateurs. Cette glace est ensuite refondue et extraite par pompe. Les seuls compresseurs de l'appareil selon le document de brevet EP0774263 correspondent à des compresseurs frigorifiques des groupes frigorifiques. Ils n'ont donc pas d'effet de compression de l'air circulant dans l'appareil selon le document de brevet EP0774263.

Il est connu du document de brevet WO2008/063252 un système de décontamination modulaire. L'air circulant dans ce système est séché en traversant une colonne contenant un gel hygroscopique. Lorsque le gel hygroscopique est saturé, il peut être régénéré (séché) par l'intermédiaire d'un dispositif spécifique. Toutefois, le système selon le document de brevet WO2008/063252 présente deux inconvénients. D'une part, la capacité d'absorption du gel hygroscopique est limitée, et une fois saturée, la colonne contenant le gel hygroscopique ne permettra plus de maintenir une humidité faible dans l'enceinte à décontaminer. D'autre part, en cas de saturation du gel hygroscopique, il convient de remplacer la colonne dont le gel hygroscopique est saturé par une autre colonne contenant du gel hygroscopique : il en résulte une opération supplémentaire chronophage et nécessite de disposer de plusieurs colonnes à gel hygroscopique en prévision d'un remplacement.

En outre, les dispositifs connus comprennent traditionnellement une ou plusieurs pompes péristaltiques pour distribuer le produit microbicide dans le dispositif en vue se son évaporation. Les pompes péristaltiques peuvent convenir pour décontaminer des enceintes d'un volume relativement important. Toutefois, elles ne permettent pas de traiter de manière satisfaisante des enceintes de faibles volumes (de l'ordre de quelques mètres cube). En effet, leur débit ne peut pas toujours être descendu à des valeurs très faibles, de l'ordre de quelques grammes par heure. Par conséquent, pour éviter un surdosage de produit microbicide dans l'enceinte à décontaminer, les pompes péristaltiques des dispositifs connus sont classiquement utilisées de façon intermittente : leur fonctionnement consiste en des arrêts et des redémarrages successifs. Il en résulte un dosage irrégulier en produit microbicide dans l'enceinte à décontaminer. De plus, le débit sortant d'une pompe péristaltique n'est pas très stable, présente une pulsation et est difficilement mesurable.

Aussi la présente invention a pour but de résoudre tout ou partie des inconvénients mentionnés ci-dessus en proposant un dispositif de décontamination aérienne par vaporisation de produit microbicide permettant de maîtriser l'humidité relative dans une enceinte fermée et présentant des coûts de fabrication, d'exploitation et de maintenance moindres.

A cet effet, la présente invention a pour objet un dispositif de décontamination aérienne mobile comprenant :
(i) un orifice d'entrée et un orifice de sortie,
(ii) des moyens de déshumidification, en aval de l'orifice d'entrée, permettant de déshumidifier l'air entrant dans le dispositif par ledit orifice d'entrée,
(iii) des moyens d'évaporation d'un produit microbicide, en amont de l'orifice de sortie et en aval des moyens de déshumidification, permettant de vaporiser le produit microbicide dans l'air circulant dans le dispositif,
caractérisé en ce que les moyens de déshumidification comprennent un compresseur volumétrique à débit constant relié à l'orifice d'entrée et destiné à la compression de l'air entrant dans le dispositif, le compresseur étant adapté pour comprimer l'air entrant dans le dispositif jusqu'à une pression prédéterminée, un condenseur à convection forcée disposé en aval du compresseur, le condenseur étant adapté pour refroidir l'air en sortie du compresseur et obtenir la condensation à température de l'ordre de 20°C de l'excédent d'eau contenu dans l'air préalablement comprimé, un filtre séparateur d'eau disposé en aval du condenseur à convection forcée pour récolter l'eau condensée, le dispositif comprenant des moyens de réglage adaptés pour faire chuter la pression de l'air filtré par le filtre jusqu'à une pression inférieure à la pression prédéterminée. Le compresseur est destiné à la compression de l'air entrant dans le dispositif. Il est adapté pour comprimer l'air entrant dans le dispositif jusqu'à une pression P prédéterminée, pour obtenir la saturation en eau de l'air entré dans le dispositif. Le refroidisseur correspond avantageusement à un condenseur à convection forcée et est adapté pour refroidir l'air en sortie du compresseur et obtenir la condensation à température ambiante de l'excédent d'eau contenu dans l'air comprimé par le compresseur. Le filtre est adapté pour récolter l'eau condensée grâce au condenseur à convection forcée. Enfin, le dispositif comprend aussi des moyens de réglage adaptés pour faire chuter la pression de l'air filtré par le filtre jusqu'à une pression sensiblement inférieure à la pression P prédéterminée, mais suffisante pour assurer la circulation dans le dispositif et dans l'enceinte à décontaminer, comme pourrait le faire un ventilateur.

Ainsi, il résulte des lois physiques qu'en comprimant de l'air ambiant et contenant de l'eau sous forme de vapeur (humidité), puis en le ramenant à une température proche de celle qu'il avait avant la compression (la compression étant accompagnée d'une hausse de la température de l'air comprimé), cet air devient sursaturé et une partie de l'eau qu'il contient va alors se condenser à température ambiante (par exemple environ 20°C). Le dispositif selon l'invention offre ainsi la possibilité de déshumidifier l'air circulant à l'intérieur du dispositif et dans l'enceinte, sans utiliser de machine frigorifique ni de produits hygroscopiques, comme c'est le cas dans les techniques traditionnelles.

Selon une autre caractéristique du dispositif de décontamination aérienne selon l'invention, les moyens d'évaporation comprennent un évaporateur comprenant un conduit débouchant sur un orifice amont et un orifice aval, lesdits moyens d'évaporation comprenant en outre des moyens de chauffe de l'évaporateur, comme une résistance électrique, permettant de chauffer l'évaporateur.

Les moyens de chauffe sont adaptés pour élever la température de l'évaporateur jusqu'à une température T prédéterminée. La température T prédéterminée est avantageusement supérieure à la température de vaporisation du produit microbicide.

Selon une possibilité, l'évaporateur correspond à un bloc métallique, le conduit étant usiné dans le bloc métallique formant l'évaporateur.

Selon un mode de réalisation, la résistance électrique est insérée dans un alésage du bloc métallique formant l'évaporateur. Selon un mode de réalisation, la sonde de température contrôlant la chauffe est insérée dans un deuxième alésage du bloc métallique formant l'évaporateur

De manière avantageuse, le dispositif de décontamination aérienne comprend des moyens d'injection du produit microbicide à l'intérieur d'un conduit des moyens d'évaporation. Selon une autre caractéristique du dispositif de décontamination aérienne selon l'invention, les moyens d'injection comprennent une aiguille d'injection présentant une première extrémité débouchant dans le conduit et une deuxième extrémité reliée à une première voie d'une vanne d'aiguillage, ladite vanne d'aiguillage comprenant une deuxième voie reliée à un corps creux destiné à contenir le produit microbicide, lesdits moyens d'injection comprenant en outre un piston apte à coulisser à l'intérieur du corps creux et un système vis-écrou actionné par un moteur pour entraîner la translation du piston dans le corps creux. Selon une possibilité, l'écrou dudit système vis-écrou est solidaire du piston.

Cela permet d'injecter le produit microbicide de façon continue à l'intérieur de l'évaporateur afin d'en faciliter l'évaporation.

Selon une autre caractéristique du dispositif de décontamination aérienne selon l'invention, la vanne d'aiguillage comprend une troisième voie destinée à être reliée à un réservoir.

De manière avantageuse, le corps creux présente un orifice de remplissage et d'expulsion relié à la deuxième voie de la vanne d'aiguillage, l'orifice de remplissage et d'expulsion étant orienté vers le haut.

Autrement dit, l'orifice de remplissage et d'expulsion est agencé dans la partie la plus haute du corps creux.

Le corps creux peut être agencé de façon sensiblement verticale.

En d'autres termes, l'orifice de remplissage et d'expulsion du corps creux est la partie la plus haute du corps creux. Ainsi, les bulles d'air qui peuvent être aspirées avec le produit microbicide lors du remplissage du corps creux par la course verticale descendante du piston ne descendent pas au fond du corps creux mais tendent à rester en partie haute du corps creux pendant son remplissage. Une course inverse du piston permet d'évacuer ces bulles d'air vers le réservoir, avant de modifier la position la position de la vanne d'aiguillage pour cette course inverse du piston permette l'expulsion du produit microbicide du corps creux en direction de l'aiguille d'injection. Ceci garantit l'absence d'air dans le produit microbicide lorsqu'il est injecté dans l'évaporateur.

Selon une forme d'exécution, le dispositif comprend des moyens d'indication de la position du piston.

Avantageusement, les moyens d'indication de la position du piston comprennent un transducteur.

Le transducteur peut correspondre à un potentiomètre linéaire dont la résistance électrique varie en fonction du déplacement du piston.

Ainsi, la combinaison du sens du mouvement du piston et de l'orientation de l'aiguillage de la vanne permet d'obtenir l'injection du produit dans l'évaporateur, ou le remplissage du corps creux en produit microbicide à partir du réservoir. La combinaison du sens du mouvement du piston et de l'orientation de la vanne d'aiguillage permet aussi de réaliser la vidange de l'aiguille d'injection vers le corps creux et la vidange du corps creux vers un réservoir.

Selon une autre caractéristique du dispositif de décontamination aérienne selon l'invention, le corps creux présente dans la partie inférieure un orifice relié à une pompe réversible et permettant de remplir le corps creux en produit microbicide à partir du réservoir, ou le vider vers le réservoir par inversion du sens.

Selon un mode de réalisation, les moyens de réglage de la pression développée par le compresseur sont agencés en aval du filtre.

Les moyens de réglage de la pression de refoulement comprennent avantageusement une vanne.

La vanne est avantageusement agencée en aval du condenseur à convection forcée et du filtre. Elle est située sur le circuit principal de circulation de l'air dans le dispositif reliant l'orifice d'entrée et l'orifice de sortie.

La vanne permet de régler la pression de refoulement du compresseur.

Selon une forme d'exécution, les moyens de réglage sont adaptés pour faire chuter la pression de l'air jusqu'à une pression proche de la pression atmosphérique.

Avantageusement, le compresseur est à débit constant.

Ainsi, il n'est pas nécessaire d'équiper le dispositif selon l'invention avec un système de régulation et des capteurs de pression, ce qui en réduit sensiblement le coût.

Selon une forme d'exécution, la pression P prédéterminée est supérieure ou égale à 3 bars, par exemple supérieure ou égale à 4 bars ou supérieure ou égale à 5 bars.

Selon une autre caractéristique du dispositif de décontamination aérienne selon l'invention, celui-ci comprend une vanne de by-pass.

La vanne de by-pass est avantageusement agencée sur un circuit secondaire s'étendant depuis l'aval du filtre jusqu'à l'orifice de sortie du dispositif.

Préférentiellement, le dispositif de décontamination aérienne selon l'invention comprend un réchauffeur d'air placé en aval du filtre séparateur d'eau et en amont de l'évaporateur, ledit réchauffeur d'air permettant d'augmenter la température de l'air en sortie du filtre séparateur d'eau.

Cette caractéristique offre l'avantage de permettre d'injecter une quantité plus importante de produit microbicide dans l'air circulant dans le dispositif que si cet air n'était pas réchauffé.

Selon une forme d'exécution, le dispositif comprend un réchauffeur d'air agencé dans le conduit, en amont du point d'injection où débouche l'aiguille d'injection, en vue d'augmenter la température de l'air avant l'injection de produit microbicide.

Avantageusement, le dispositif de décontamination aérienne selon l'invention comprend un réservoir en coude relié à l'orifice aval d'une part et à l'orifice de sortie d'autre part.

Cette caractéristique permet de collecter les gouttelettes en sortie de l'évaporateur qui se formeraient dans le cas d'un dysfonctionnement du système, empêchant ainsi l'envoi dans l'enceinte de produit microbicide liquide non évaporé et donc très corrosif.

Selon une autre caractéristique du dispositif de décontamination aérienne selon l'invention, le refroidisseur ou condenseur à convection forcée comprend un tube métallique et un ventilateur permettant de refroidir le tube métallique en propulsant de l'air ambiant en direction dudit tube métallique.

Avantageusement, le dispositif comprend des moyens de refroidissement complémentaire destinés à refroidir l'air en sortie du compresseur en complément du condenseur à convection forcée.

Par exemple, les moyens de refroidissement complémentaire comprennent un module à effet Peltier.

Selon encore une autre caractéristique du dispositif de décontamination aérienne selon l'invention le refroidisseur comprend une machine frigorifique.

Cette caractéristique offre la possibilité de d'abaisser la température de l'air de manière significative, jusqu'à des températures inférieures à celle de l'air ambiant, par exemple une température égale ou inférieure à 5°C.

Avantageusement, le dispositif de décontamination aérienne selon l'invention comprend des roulettes.

Préférentiellement, le dispositif de décontamination aérienne selon l'invention comprend deux flexibles de raccordement à l'enceinte.

Cette caractéristique offre l'avantage de permettre d'utiliser le dispositif selon l'invention à l'extérieur de l'enceinte, ce qui permet d'intervenir sur le dispositif sans entrer dans l'enceinte dont l'air contient du produit microbicide.

Avantageusement, le produit microbicide est un liquide contenant du peroxyde d'hydrogène et de l'eau.

Selon encore une autre caractéristique du dispositif de décontamination aérienne selon l'invention, celui-ci comporte un catalyseur placé en aval du compresseur.

L'invention sera mieux comprise à l'aide de la description détaillée d'un mode de réalisation de l'invention qui est exposée ci-dessous en regard des dessins annexés dans lesquels :
- La figure 1 représente une vue schématique en coupe et de profil d'un dispositif de décontamination aérienne selon un mode particulier de réalisation de l'invention,
- La figure 2 montre une vue schématique en coupe et de profil d'un dispositif de décontamination aérienne selon un autre mode particulier de réalisation de l'invention,
- La figure 3 est une vue schématique en coupe et de profil d'un dispositif de décontamination aérienne selon un autre mode de réalisation de l'invention.

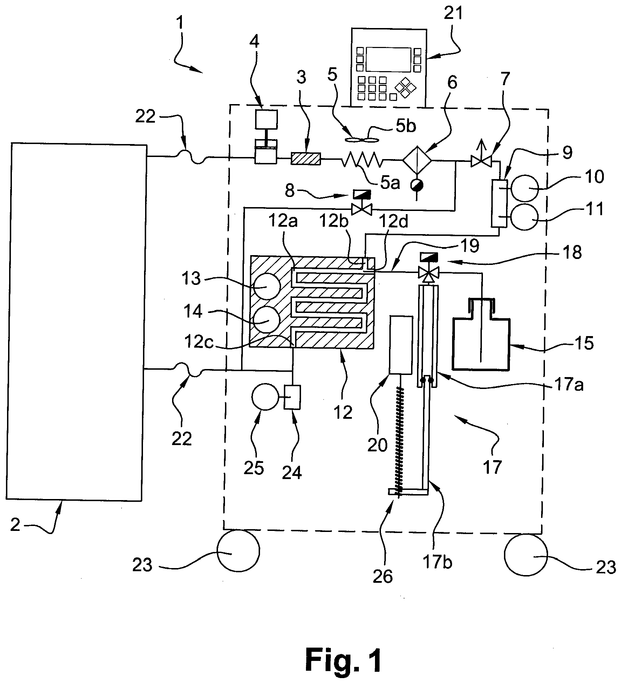

La figure 1 montre de façon schématique un dispositif 1 de décontamination aérienne selon un mode particulier de réalisation de la présente invention. Le dispositif 1 de décontamination aérienne est destiné à décontaminer, par vaporisation d'un produit microbicide tel qu'un liquide contenant du peroxyde d'hydrogène, l'air contenu dans une enceinte 2 fermée. De manière subsidiaire, le dispositif 1 de décontamination aérienne permet de décontaminer la surface des objets présents à l'intérieur de l'enceinte 2. Le cycle de décontamination peut comporter une phase de décontamination proprement dite pendant laquelle le produit microbicide est vaporisé et injecté dans l'enceinte 2 suivie d'une phase de diminution progressive de la teneur en peroxyde d'hydrogène contenu dans l'air de l'enceinte 2.

Le dispositif 1 comprend un orifice d'entrée et un orifice de sortie permettant respectivement l'admission de l'air contenu dans l'enceinte 2 à l'intérieur du dispositif 1 et l'expulsion de cet air hors du dispositif 1, vers l'intérieur de l'enceinte 2.

Comme cela est représenté sur les figures 1 à 3, le dispositif 1 comprend des moyens de déshumidification de l'air. Ces moyens de déshumidification sont notamment formés par un compresseur 4, un refroidisseur et un filtre 6.

Le refroidisseur correspond avantageusement à un condenseur 5 à convection forcée, comme cela est illustré sur les figures 1 à 3.

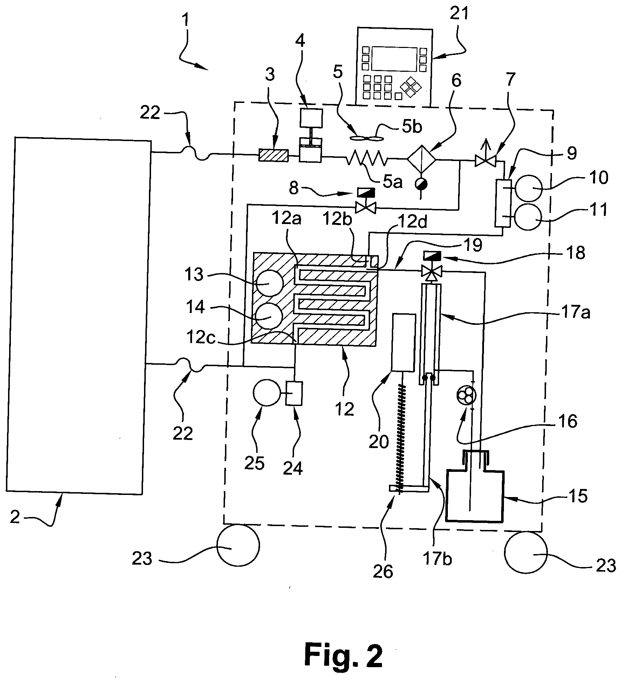

Le compresseur 4 assure la circulation de l'air à l'intérieur du dispositif 1, et donc de l'enceinte 2 du fait du système en circuit fermé. Il aspire l'air en amont qu'il refoule en aval, vers un catalyseur 3 installé en sortie et vers le refroidisseur. Amont et aval sont définis par rapport au sens de circulation de l'air à l'intérieur du dispositif 1. L'air sortant du compresseur 4 et entrant ensuite dans le catalyseur 3 présente une pression et une température plus élevées qu'en amont, du fait de la compression. Cette température élevée accélère avantageusement la dismutation du peroxyde d'hydrogène dans le catalyseur 3, permettant de le décomposer en eau et en dioxygène. Dans une variante de réalisation, le catalyseur 3 peut également être relié à l'orifice d'entrée du dispositif 1, c'est-à-dire être placé en amont du compresseur 4 au lieu d'être placé en aval, comme cela est visible sur la figure 2.

La pression en sortie du compresseur 4 est avantageusement supérieure à 3 bars, 4 bars ou 5 bars.

Le compresseur 4 est agencé sur la trajectoire de l'air entrant dans le dispositif 1 : l'air entrant dans le dispositif 1 passe nécessairement par le compresseur 4. Le compresseur 4 est en effet placé sur le circuit principal de circulation d'air dans le dispositif 1.

Le compresseur 4 est avantageusement un compresseur volumétrique à débit constant. Il n'y a donc pas besoin de prévoir des capteurs de mesure de débit et un système de régulation du débit d'air circulant dans le dispositif 1, ce qui contribue à la réduction des coûts.

Pendant la phase de décontamination, l'utilisation du catalyseur 3 peut permettre de contrôler au fur et à mesure du cycle de décontamination la teneur en peroxyde d'hydrogène dans l'air. A l'issue de la phase de décontamination, l'utilisation du catalyseur 3 permet de diminuer progressivement la teneur en peroxyde d'hydrogène de l'air circulant dans le dispositif 1 et par conséquent celle de l'air de l'enceinte 2.

Le refroidisseur ou condenseur 5 à convection forcée peut comporter un tube 5a métallique, par exemple en aluminium ou en acier inoxydable, afin de résister à la corrosion par le peroxyde d'hydrogène résiduel qui peut subsister en sortie du catalyseur 3. Le refroidisseur ou condenseur à convection forcée peut comporter en outre un ventilateur 5b permettant le refroidissement du tube 5a métallique avec l'air ambiant.

Le condenseur 5 à convection forcée est agencé pour que l'air en sortie du compresseur 4 circule dans le tube 5a métallique.

Le ventilateur 5b est agencé pour générer un flux d'air ambiant en direction du tube 5a métallique.

Le refroidisseur peut aussi comprendre une machine frigorifique, par exemple une machine à absorption, permettant de diminuer la température de l'air comprimé jusqu'à des valeurs inférieures ou égales à 5°C.

Selon un mode de réalisation, le dispositif 1 peut comprendre un module 27 à effet Peltier en complément du condenseur 5 à convection forcée.

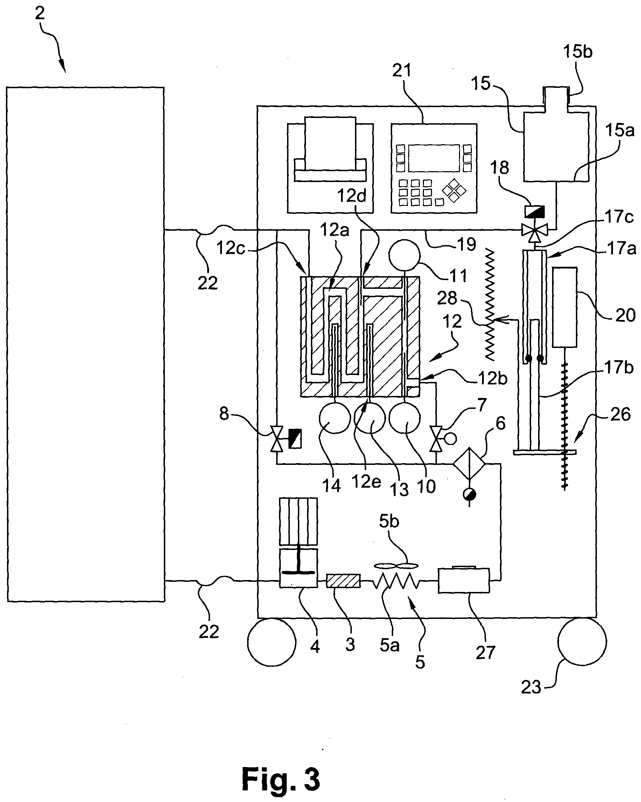

Comme cela est visible sur la figure 3, le module 27 à effet Peltier est agencé en aval du condenseur 5 à convection forcée, et en amont du filtre 6 séparateur d'eau. Le module 27 à effet Peltier permet de diminuer encore plus la quantité d'eau contenue dans l'air comprimé, en le refroidissant davantage, par exemple jusqu'à une température proche de 0°C.

Sous l'effet du refroidissement, l'air comprimé se sature en eau. L'excédent d'eau qu'il contenait initialement mais qu'il ne peut désormais plus contenir sous forme gazeuse se condense alors ; des gouttelettes d'eau se forment.

Ces gouttelettes sont filtrées au moyen du filtre 6 séparateur d'eau. Le filtre 6 séparateur d'eau est placé en aval du refroidisseur ou condenseur à convection forcée 5, et le cas échéant du module 27 à effet Peltier. Le filtre 6 peut permettre de filtrer des gouttelettes de diamètre de l'ordre de quelques microns. Une purge automatique du filtre 6 peut être prévue pour permettre l'évacuation de l'eau amassée par le filtre 6. En sortie du filtre 6, l'air est détendu. Son volume augmente. La quantité d'eau qu'il contenait a été réduite. Son humidité relative est ainsi diminuée.

On notera que le filtre 6 séparateur d'eau à purge automatique est installé en aval du condenseur 5 à convection forcée et permet l'évacuation automatique de l'eau sans devoir utiliser une pompe d'extraction.

A titre d'exemple, l'air de l'enceinte 2 dont la température est de 20°C et l'humidité relative est de 60%, contient 9 g d'eau par kilogramme d'air. Aspiré et comprimé à 5 bars par le compresseur 4, la température de cet air aspiré et comprimé va s'élever jusqu'à une valeur de l'ordre de 150°C. Cette température élevée accélère la dismutation du peroxyde d'hydrogène dans le catalyseur 3. Cet air comprimé, refroidi ensuite jusqu'à une température de l'ordre de 20°C par le refroidisseur ou condenseur 5 à convection forcée, est sursaturé et ne peut alors contenir plus que 2,7 g d'eau par kilogramme d'air au lieu de 9 g d'eau par kilogramme d'air (se reporter au tableau ci-après). Son humidité relative est alors de 100%. Le filtre 6 permet d'éliminer l'excédent d'eau, condensé sous forme de gouttelettes. L'air en sortie du filtre 6, contenant 2,7 g d'eau par kilogramme d'air, est ensuite détendu ; son volume augmente. Son humidité relative sera alors de 17%, alors qu'elle était initialement de 60%.

Pour obtenir la même hygrométrie de 17% sans compression, il serait nécessaire de le refroidir à -5°C au lieu de 20°C, autrement dit de transformer l'eau qu'il contient en glace. Le dispositif 1 selon l'invention permet de s'en affranchir.

Si un module 27 à effet Peltier est installé et ramène la température de l'air comprimé à une température de l'ordre de 0°C, celui-ci ne peut alors contenir plus que 0,6 g d'eau par kilogramme d'air au lieu de 9 g d'eau par kilogramme d'air. Son humidité relative (en ramenant la température à 20°C) est alors de 4%, alors qu'elle était initialement de 60%. Pour obtenir la même hygrométrie de 4% sans compression, il serait nécessaire de le refroidir à environ -25°C au lieu de 0°C. Il peut également être envisagé d'utiliser un gel hygroscopique.

Le tableau comparatif suivant montre la différence de résultat obtenu en termes de quantité d'eau résiduelle contenue dans l'air lorsque celui-ci est refroidi jusqu'à une température T sans avoir été préalablement comprimé (cas d'un dispositif dont la circulation de l'air est assuré par un ventilateur uniquement), et lorsque celui-ci est refroidi après avoir été comprimé jusqu'à 5 bars (comme c'est le cas avec le dispositif 1, grâce au compresseur 4) :

| T | 20°C | 10°C | 0°C |
|---|---|---|---|
| Air refroidi sans le comprimer | 9 g/kg | 7,5 g/kg | 3,8 g/kg |
| Air refroidi après compression à 5 b | 2,7 g/kg | 1,2 g/kg | 0,6 g/kg |

Il ressort clairement du tableau ci-dessus que la déshumidification de l'air circulant dans le dispositif 1 est sensiblement plus importante (quantité d'eau résiduelle moindre) lorsque l'air a été comprimé par le compresseur 4 avant d'avoir été refroidi. On voit donc l'intérêt de la compression de l'air entrant dans le dispositif 1 pour déshumidifier efficacement cet air.

Comme cela est visible sur les figures 1 à 3, il est possible de prévoir des moyens de réglage de la pression de refoulement du compresseur 4, comme une vanne 7 à pointeau placée en aval du filtre 6. Cela permet à un utilisateur du dispositif 1 de sélectionner le taux d'humidité résiduel de l'air en sortie du filtre 6.

La vanne 7 est avantageusement placée en aval du filtre 6, pour détendre l'air préalablement comprimé, refroidi et filtré. On notera que la pression en sortie de la vanne 7 peut être proche de la pression atmosphérique, mais suffisante pour assurer la circulation dans le dispositif et dans l'enceinte à décontaminer.

Il est également possible de prévoir une vanne 8 automatique de by-pass, également placée en aval du filtre 6, en parallèle avec la vanne 7 à pointeau. La vanne 8 de by-pass est destinée à s'ouvrir à l'issue de la phase de décontamination afin de permettre de faire chuter la pression en sortie du compresseur 4. Le débit d'air aspiré puis refoulé par le compresseur 4 est ainsi augmenté. Cela permet d'accélérer la circulation de l'air à l'intérieur du dispositif 1, en particulier dans le catalyseur 3. La teneur en peroxyde d'hydrogène à l'intérieur de l'air contenu dans l'enceinte 2 est ainsi rapidement réduite.

Comme cela est visible sur les figures 1 à 3, la vanne 8 de by-pass est agencée sur un circuit secondaire (de by-pass) s'étendant depuis l'aval du filtre 6 jusqu'à l'orifice de sortie du dispositif 1.

En aval de la vanne 7 peut être prévu un réchauffeur d'air 9 comprenant par exemple une résistance électrique 10 et une sonde de température 11 permettant de contrôler la température du réchauffeur d'air 9. Ce réchauffeur d'air 9 permet de chauffer l'air déshumidifié en vue d'augmenter la quantité de peroxyde d'hydrogène qui va pouvoir être injecté dans cet air sans qu'il soit saturé.

Le dispositif 1 peut comprendre un réservoir 15 ou être destiné à être relié à un tel réservoir permettant de stocker le produit microbicide. Le produit microbicide est avantageusement un liquide contenant 35% de peroxyde d'hydrogène et 65% d'eau.

Le dispositif 1 comprend des moyens d'évaporation du produit microbicide, formés par exemple par un évaporateur 12. L'évaporateur 12 est une pièce métallique, par exemple en aluminium ou en acier inoxydable. Il comprend un conduit 12a muni d'un orifice amont 12b d'entrée et d'un orifice aval 12c de sortie. De manière avantageuse, le conduit 12a présente un diamètre calculé pour obtenir une grande vitesse de l'air, et présente également une ou plusieurs chicanes. Dans le mode de réalisation décrit, l'évaporateur 12 comprend une résistance électrique 13 destinée à le chauffer à une température supérieure à la température d'ébullition du produit microbicide, de façon à ce que tout dépôt de celui-ci s'évapore immédiatement. Il comprend également une sonde de température 14 permettant de contrôler cette température. Pour un liquide contenant 35% de peroxyde d'hydrogène et 65% d'eau, la température de l'évaporateur 12 peut être par exemple maintenue à 110°C.

L'évaporateur 12 peut avantageusement se présenter sous la forme d'un bloc métallique dans lequel est usiné le conduit 12a.

La résistance électrique 13 peut se présenter sous la forme d'une cartouche cylindrique insérée dans un alésage 12e du bloc métallique formant l'évaporateur 12. La sonde de température 14 peut aussi être insérée dans cet alésage 12e (ou dans un alésage distinct) en vue de la régulation de la température de la résistance électrique 13.

Le dispositif 1 comprend aussi des moyens d'injection du produit microbicide à l'intérieur de l'évaporateur 12. Ces moyens d'injection comprennent une aiguille d'injection 19, une vanne 18 d'aiguillage, un corps creux 17a longitudinal dans lequel est destiné à coulisser un piston 17b, un moteur 20, et un système vis-écrou 26. Le corps creux 17a peut être à cavité cylindrique, et le piston 17b peut être cylindrique également.

Le moteur 20 peut fonctionner dans les deux sens de rotation, pour déplacer le piston 17b dans un sens ou dans l'autre, et peut aussi fonctionner à vitesse variable.

L'aiguille d'injection 19 dont le passage intérieur du produit est très réduit de façon à avoir une vitesse importante même avec un débit de produit très faible, présente une première extrémité débouchant dans le conduit 12a et une deuxième extrémité reliée à la vanne 18 d'aiguillage.

La première extrémité de l'aiguille d'injection 19 débouche dans le conduit 12a au niveau du point d'injection 12d.

Les chicanes du conduit 12a vont favoriser le dépôt de produit encore liquide et favoriser son évaporation. La grande vitesse de l'air, engendrée par le débit du compresseur 4 et le diamètre du conduit 12a, va faciliter l'arrachement du produit microbicide dès sa sortie de l'aiguille d'injection 19.

Le conduit 12a usiné peut comprendre selon la figure 3, dans sa partie amont au point d'injection 12d, le réchauffeur d'air 9. Ce dernier peut se présenter sous la forme d'une cartouche insérée dans l'axe du conduit 12a avec un système d'étanchéité. Le réchauffeur d'air 9 permet de chauffer l'air préalablement déshumidifié en vue d'augmenter la quantité de produit microbicide qui va pouvoir être injecté dans cet air sans le saturer.

Ainsi, le conduit 12a usiné peut comprendre, dans sa partie amont au point d'injection 12d, la résistance électrique 10 et la sonde de température 11. La partie amont au point d'injection 12d du conduit 12a peut donc être conformée pour recevoir la résistance électrique 10 et le cas échéant la sonde de température 11. Elle peut être sensiblement rectiligne. Ainsi, le conduit 12a usiné peut comprendre dans sa partie amont au point d'injection 12d le réchauffeur d'air 9 sous forme d'une cartouche insérée dans l'axe du conduit 12a.

A titre d'exemple, pour un compresseur 4 délivrant 2 m³/heure d'air, le diamètre du conduit 12a de l'évaporateur 12 peut être de 5 mm, ce qui donne une vitesse d'air de 30 m/s. Le débit de produit microbicide en sortie de l'aiguille d'injection 19 nécessaire au procédé de décontamination peut alors être ajusté à 40 ml/h (0,04 litres/heure), ce qui est très faible, mais la vitesse de passage du produit microbicide dans l'aiguille d'injection 19 (dont le diamètre intérieur est de 0,13 mm) sera de 0,8 m/s. Le produit microbicide sort ainsi de l'aiguille d'injection 19 de façon continue et non en goutte à goutte.

Le produit microbicide parvient à l'extrémité de l'aiguille d'injection 19 débouchant dans le conduit 12a. Ce produit est arraché par le flux d'air chaud circulant dans le conduit 12a, de sorte qu'il se vaporise au moins partiellement par entraînement. Les gouttelettes résiduelles sont projetées sur les parois du conduit 12a, ce qui est favorisé par la grande vitesse de circulation de l'air et l'existence des chicanes. Elles se vaporisent alors au contact de celles-ci.

Le piston 17b est apte à coulisser à l'intérieur du corps creux 17a. L'étanchéité entre le piston 17b et le corps creux 17a peut être assurée par un joint torique. Le piston 17b est lié à l'écrou d'un système vis-écrou 26. La vis du système vis-écrou est quant à elle entraînée en rotation par un moteur 20. Ainsi, le moteur 20 entraîne la rotation de la vis, qui elle-même provoque la translation du piston 17b dans le corps creux 17a. Le moteur 20 peut être électrique, à vitesse variable, sa vitesse et son sens de rotation étant contrôlés par une consigne.

La vanne 18 d'aiguillage communique également avec le réservoir 15. Le corps creux 17a présente à son point le plus haut un orifice relié à une des trois voies de la vanne 18 d'aiguillage.

Selon un mode de réalisation, le réservoir 15 est avantageusement disposé au dessus de la vanne 18 d'aiguillage de façon à faciliter l'écoulement par gravité du produit microbicide vers la vanne 18 d'aiguillage et le corps creux 17a.

Comme cela est illustré à la figure 3, le fond 15a du réservoir 15 est relié à la vanne 18 d'aiguillage.

On remarquera en outre que le goulot 15b du réservoir 15 débouche à l'extérieur du dispositif 1 pour faciliter l'introduction de produit microbicide dans le réservoir 15.

Pendant le remplissage du corps creux 17a, la vanne 18 d'aiguillage oriente le produit microbicide depuis le réservoir 15 vers l'intérieur du corps creux 17a, le transfert du liquide contenant le peroxyde d'hydrogène étant assuré par un mouvement de translation du piston inversé par rapport à celui assurant l'injection.

Avant d'injecter le produit, le mouvement du piston 17b peut être inversé provisoirement sans inverser la position de la vanne 18 de façon à évacuer vers le réservoir les bulles d'air éventuellement présentes, et qui vont se maintenir en partie haute pendant et après le remplissage. Ainsi, le produit microbicide injecté ensuite dans l'évaporateur 12 est exempt de bulles d'air

Pendant l'injection du produit microbicide à l'intérieur de l'évaporateur 12, la vanne 18 d'aiguillage oriente ce produit vers l'aiguille d'injection 19. Le produit microbicide est alors progressivement expulsé hors du corps creux 17a grâce à la translation du piston 17b.

En fin de cycle, il est possible de vidanger l'ensemble du circuit en orientant l'expulsion du produit microbicide vers le réservoir 15 par l'inversion de la vanne 18 d'aiguillage, de façon à empêcher la stagnation du produit entre deux cycles, et en vue de faciliter les opérations éventuelles de maintenance. Il sera également possible de vidanger l'aiguille d'injection 19 vers l'intérieur du corps creux 17a en inversant simultanément le sens de translation du piston 17b et l'orientation de la vanne 18 d'aiguillage.

Le sens de mouvement du piston 17b et l'orientation de l'aiguillage de la vanne 18 permettent d'obtenir, selon les combinaisons: l'injection du produit microbicide dans l'évaporateur 12, le remplissage du corps creux 17a du vérin 17 en produit microbicide à partir du réservoir 15, la vidange de l'aiguille d'injection 19 vers le corps creux 17a du vérin 17, et la vidange du vérin 17 dans le réservoir 15.

Selon une variante de réalisation, visible sur la figure 2, le corps creux 17a peut également comprendre dans la partie inférieure un orifice relié à une pompe 16 réversible, elle-même reliée au réservoir 15. La troisième voie de la vanne 18 est alors reliée à la partie haute du réservoir 15. Ce dispositif permet de remplir le corps creux en produit microbicide à partir du réservoir, ou le vider vers le réservoir par inversion du sens.

Les moyens d'injection permettent avantageusement d'obtenir un débit très faible de produit microbicide dans l'évaporateur 12, de sorte que ce débit est constant y compris pour traiter des enceintes de faible volume. Par exemple, pour une enceinte à décontaminer correspondant à un passe-plat d'un volume de 0,125 m³ (volume cubique dont l'arête est de 50 cm par exemple) dans laquelle on souhaite obtenir une concentration de 500 ppm en produit microbicide avec un débit d'air circulant dans le dispositif de 2m³/h (par l'intermédiaire du compresseur 4), le débit de produit microbicide injecté dans l'évaporateur 12 doit être approximativement de 4 g/h. Les dispositifs traditionnels généralement équipés de pompes péristaltiques ne permettent pas de parvenir à un débit aussi faible.

Un système de contrôle et commande 21 est prévu pour gérer le fonctionnement du dispositif 1 pendant les cycles successifs de décontamination et de diminution de la teneur en peroxyde d'hydrogène.

Selon une possibilité illustrée sur la figure 3, le dispositif 1 comprend des moyens d'indication de la position du piston 17b pour en déduire à chaque instant le volume, donc la quantité de produit microbicide injecté dans l'évaporateur 12. Les moyens d'indication de la position du piston 17b peuvent notamment comprendre un transducteur 28. Le transducteur 28 permet avantageusement de transformer une grandeur représentative de la position du piston 17b en, par exemple, un signal électrique proportionnel à la distance entre la position du piston 17b à un instant t et l'une des deux positions extrêmes qu'il peut adopter. Le transducteur 28 peut correspondre à un potentiomètre linéaire, dont la résistance électrique peut être proportionnelle à la distance en question.

Le dispositif 1 peut en outre comporter des moyens de raccordement, par exemple des flexibles 22. Une première extrémité d'un des flexibles 22 est raccordée à l'orifice d'entrée (ou de sortie) du dispositif 1. Une deuxième extrémité de ce flexible 22 débouche à l'intérieur de l'enceinte 2.

Sur la liaison flexible ramenant l'air de l'enceinte 2 vers le dispositif 1, il est possible d'installer un dispositif de mesure de la teneur en peroxyde d'hydrogène extrait de l'enceinte 2 afin de vérifier que le cycle s'est déroulé correctement et que la teneur de l'air en peroxyde d'hydrogène a été réduite jusqu'à une valeur acceptable, c'est-à-dire une valeur pour laquelle il n'existe aucun risque pour une personne entrant dans l'enceinte 2, à l'issue du cycle de décontamination.

Le dispositif 1 peut également être équipé de moyens de roulement, par exemple des roulettes 23.

Le dispositif 1 peut comporter un réservoir 24 en coude, placé en sortie de l'évaporateur 12. Le réservoir 24 en coude permet de collecter les gouttelettes qui se formeraient dans le cas d'un dysfonctionnement du système, empêchant ainsi l'envoi dans l'enceinte de produit microbicide liquide non évaporé et donc très corrosif. Une sonde de niveau 25 peut être adjointe au réservoir 24 en coude pour alerter un utilisateur de l'existence d'un tel dysfonctionnement.

Bien que l'invention ait été décrite en liaison avec des exemples particuliers de réalisation, il est bien évident qu'elle n'y est nullement limitée et qu'elle comprend tous les équivalents techniques des moyens décrits ainsi que leurs combinaisons si celles-ci entrent dans le cadre de l'invention.

## Revendications

1. Dispositif (1) de décontamination aérienne mobile comprenant :
(i) un orifice d'entrée et un orifice de sortie,
(ii) des moyens de déshumidification, en aval de l'orifice d'entrée, permettant de déshumidifier l'air entrant dans le dispositif (1) par ledit orifice d'entrée,
(iii) des moyens d'évaporation d'un produit microbicide, en amont de l'orifice de sortie et en aval des moyens de déshumidification, permettant de vaporiser le produit microbicide dans l'air circulant dans le dispositif (1),
**caractérisé en ce que** les moyens de déshumidification comprennent un compresseur (4) volumétrique à débit constant relié à l'orifice d'entrée et destiné à la compression de l'air entrant dans le dispositif (1), le compresseur (4) étant adapté pour comprimer l'air entrant dans le dispositif (1) jusqu'à une pression (P) prédéterminée, un condenseur (5) à convection forcée disposé en aval du compresseur (4), le condenseur (5) étant adapté pour refroidir l'air en sortie du compresseur (4) et obtenir la condensation à température de l'ordre de 20°C de l'excédent d'eau contenu dans l'air préalablement comprimé, un filtre (6) séparateur d'eau disposé en aval du condenseur (5) à convection forcée pour récolter l'eau condensée, le dispositif (1) comprenant des moyens de réglage adaptés pour faire chuter la pression de l'air filtré par le filtre (6) jusqu'à une pression inférieure à la pression (P) prédéterminée.

2. Dispositif (1) de décontamination aérienne selon la revendication 1, **caractérisé en ce qu'**il comprend des moyens d'injection du produit microbicide à l'intérieur d'un conduit (12a) des moyens d'évaporation, les moyens d'injection comprenant une aiguille d'injection (19) présentant une première extrémité débouchant dans le conduit (12a) et une deuxième extrémité communiquant avec un corps creux (17a) destiné à contenir le produit microbicide, lesdits moyens d'injection comprenant en outre un piston (17b) apte à coulisser à l'intérieur du corps creux (17a) et un système vis-écrou (26) actionné par un moteur (20) pour entraîner la translation du piston (17b) dans le corps creux (17a).

3. Dispositif (1) de décontamination aérienne selon la revendication 2, **caractérisé en ce que** le dispositif comprend une vanne (18) d'aiguillage comportant trois voies reliée par une première voie à la deuxième extrémité de l'aiguille d'injection (19), par une deuxième voie à un orifice (17c) d'expulsion situé au point le plus haut du corps creux (17a), et par une troisième voie à un réservoir (15).

4. Dispositif (1) de décontamination aérienne selon la revendication 2 ou 3, **caractérisé en ce que** le dispositif (1) comprend des moyens d'indication de la position du piston (17b).

5. Dispositif (1) décontamination aérienne selon la revendication 2, **caractérisé en ce que** le corps creux (17a) présente au point le plus bas un orifice de remplissage et de vidange relié à une pompe réversible (16) permettant de remplir ou vidanger le corps creux (17a) en produit microbicide à partir du réservoir (15).

6. Dispositif (1) de décontamination aérienne selon l'une des revendications 1 à 5, **caractérisé en ce que** les moyens d'évaporation comprennent un évaporateur (12) comprenant le conduit (12a) débouchant sur un orifice amont (12b) et un orifice aval (12c), lesdits moyens d'évaporation comprenant en outre des moyens de chauffe permettant de chauffer l'évaporateur (12), l'évaporateur (12) correspondant à un bloc métallique et le conduit (12a) étant usiné dans le bloc métallique formant l'évaporateur (12).

7. Dispositif (1) de décontamination aérienne selon la revendication 6, **caractérisé en ce que** les moyens de chauffe comprennent une résistance électrique (13) insérée dans un alésage (12e) du bloc métallique formant l'évaporateur (12).

8. Dispositif (1) de décontamination aérienne selon l'une des revendications 2 à 7, **caractérisé en ce que** le dispositif (1) comprend un réchauffeur d'air (9) agencé dans le conduit (12a), en amont d'un point d'injection (12d) où débouche l'aiguille d'injection (19), en vue d'augmenter la température de l'air avant l'injection de produit microbicide.

9. Dispositif (1) de décontamination aérienne selon l'une des revendications 1 à 8, **caractérisé en ce que** les moyens de réglage de la pression de refoulement comprennent une vanne (7).

10. Dispositif (1) de décontamination aérienne selon l'une des revendications 1 à 9, **caractérisé en ce que** la pression (P) prédéterminée est supérieure ou égale à 3 bars (300kPa).

11. Dispositif (1) de décontamination aérienne selon l'une des revendications 1 à 10, **caractérisé en ce que** le condenseur (5) à convection forcée (5) comprend un tube (5a) métallique et un ventilateur (5b) permettant de refroidir le tube (5a) métallique en propulsant de l'air ambiant en direction dudit tube (5a) métallique.

12. Dispositif (1) de décontamination aérienne selon l'une des revendications 1 à 11, **caractérisé en ce que** le dispositif (1) des moyens de refroidissement complémentaire destinés à refroidir l'air en sortie du compresseur (4) en complément du condenseur (5) à convection forcée.

13. Dispositif (1) de décontamination aérienne selon l'une des revendications 1 à 12, **caractérisé en ce qu'**il comprend une vanne de by-pass (8).

14. Dispositif (1) de décontamination aérienne selon l'une des revendications 1 à 12, **caractérisé en ce qu'**il comporte un catalyseur (3) placé en amont du compresseur (4).

## Patentansprüche

1. Mobile Luftdekontaminationsvorrichtung (1), Folgendes umfassend:
(i) eine Eingangsöffnung und eine Ausgangsöffnung,
(ii) Entfeuchtungsmittel, stromabwärts der Eingangsöffnung, die es ermöglichen, die durch die besagte Eingangsöffnung in die Vorrichtung (1) eingehende Luft zu entfeuchten,
(iii) Mittel zum Verdampfen eines mikrobiziden Mittels, stromaufwärts der Ausgangsöffnung und stromabwärts der Entfeuchtungsmittel, die es ermöglichen, das mikrobizide Mittel in die Luft zu zerstäuben, die in der Vorrichtung (1) zirkuliert,
**dadurch gekennzeichnet, dass** die Entfeuchtungsmittel einen volumetrischen Kompressor (4) mit konstantem Volumenstrom umfassen, der mit der Eingangsöffnung verbunden ist und zur Verdichtung der in die Vorrichtung (1) eingehenden Luft bestimmt ist, wobei der Kompressor (4) ausgeführt ist, um die in die Vorrichtung (1) eingehende Luft bis auf einen vorbestimmten Druck (P) zu verdichten, einen Kondensator (5) mit Zwangskonvektion, der stromabwärts des Kompressors (4) angeordnet ist, wobei der Kondensator (5) ausgeführt ist, um die Luft am Ausgang des Kompressors (4) abzukühlen, und um die Kondensation auf einer Temperatur in der Größenordnung von 20 °C des überschüssigen Wassers zu erhalten, das in der zuvor komprimierten Luft enthalten ist, einen Wasserabscheider-Filter (6), der stromabwärts des Kondensators (5) mit Zwangskonvektion angeordnet ist, um das kondensierte Wasser aufzufangen, wobei die Vorrichtung (1) Einstellmittel umfasst, die ausgeführt sind, um den Druck der durch den Filter (6) gefilterten Luft bis auf einen Druck unter dem vorbestimmten Druck (P) abfallen zu lassen.

2. Luftdekontaminationsvorrichtung (1) nach Anspruch 1, **dadurch gekennzeichnet, dass** sie Mittel zum Injizieren des mikrobiziden Mittels ins Innere einer Leitung (12a) der Mittel zum Verdampfen umfasst, wobei die Mittel zum Injizieren eine Injektionsnadel (19) umfassen, die ein erstes Ende aufweist, das in die Leitung (12a) mündet, und ein zweites Ende, das mit einem Hohlkörper (17a) kommuniziert, der dazu bestimmt ist, das mikrobizide Mittel zu enthalten, wobei die besagten Mittel zum Injizieren darüber hinaus einen Kolben (17b) umfassen, der imstande ist, im Inneren des Hohlkörpers (17a) zu gleiten, sowie ein Spindel-Mutter-System (26), das durch einen Motor (20) betätigt wird, um die Vorschubbewegung des Kolbens (17b) im Hohlkörper (17a) anzutreiben.

3. Luftdekontaminationsvorrichtung (1) nach Anspruch 2, **dadurch gekennzeichnet, dass** die Vorrichtung ein Verteilerventil (18) enthält, das drei Wege umfasst, und über einen ersten Weg mit dem zweiten Ende der Injektionsnadel (19), über einen zweiten Weg mit einer Ausstoßöffnung (17c), die sich am höchsten Punkt des Hohlkörpers (17a) befindet, und über einen dritten Weg mit einem Behälter (15) verbunden ist.

4. Luftdekontaminationsvorrichtung (1) nach Anspruch 2 oder 3, **dadurch gekennzeichnet, dass** die Vorrichtung (1) Mittel zum Anzeigen der Position des Kolbens (17b) umfasst.

5. Luftdekontaminationsvorrichtung (1) nach Anspruch 2, **dadurch gekennzeichnet, dass** der Hohlkörper (17a) am niedrigsten Punkt eine Füll- und Ablassöffnung umfasst, die mit einer umkehrbaren Pumpe (16) verbunden ist, die es ermöglicht, den Hohlkörper (17a) mit dem mikrobiziden Mittel aus dem Behälter (15) zu füllen, oder ihn zu entleeren.

6. Luftdekontaminationsvorrichtung (1) nach einem der Ansprüche 1 bis 5, **dadurch gekennzeichnet, dass** die Mittel zum Verdampfen einen Verdampfer (12) umfassen, der die Leitung (12a) umfasst, die in eine Eintrittsöffnung (12b) und eine Austrittsöffnung (12c) mündet, wobei die besagten Mittel zum Verdampfen darüber hinaus Heizmittel umfassen, die es ermöglichen, den Verdampfer (12) zu erhitzen, wobei der Verdampfer (12) einem Metallblock entspricht, und die Leitung (12a) in den Metallblock eingearbeitet ist, der den Verdampfer (12) bildet.

7. Luftdekontaminationsvorrichtung (1) nach Anspruch 6, **dadurch gekennzeichnet, dass** die Heizmittel einen elektrischen Widerstand (13) umfassen, der in eine Bohrung (12e) des Metallblocks eingeführt wird, der den Verdampfer (12) bildet.

8. Luftdekontaminationsvorrichtung (1) nach einem der Ansprüche 2 bis 7, **dadurch gekennzeichnet, dass** die Vorrichtung (1) einen Lufterhitzer (9) umfasst, der stromaufwärts einer Injektionsstelle (12d), in welche die Injektionsnadel (19) mündet, in der Leitung (12a) angeordnet ist, um die Temperatur der Luft vor der Injektion des mikrobiziden Mittels zu erhöhen.

9. Luftdekontaminationsvorrichtung (1) nach einem der Ansprüche 1 bis 8, **dadurch gekennzeichnet, dass** die Einstellmittel für den Förderdruck ein Ventil (7) umfassen.

10. Luftdekontaminationsvorrichtung (1) nach einem der Ansprüche 1 bis 9, **dadurch gekennzeichnet, dass** der vorbestimmte Druck (P) größer oder gleich 3 bar (300 kPa) ist.

11. Luftdekontaminationsvorrichtung (1) nach einem der Ansprüche 1 bis 10, **dadurch gekennzeichnet, dass** der Kondensator (5) mit Zwangskonvektion (5) eine Metallröhre (5a) und einen Lüfter (5b) umfasst, der es ermöglicht, die Metallröhre (5a) abzukühlen, indem er Umgebungsluft in Richtung der besagten Metallröhre (5a) bläst.

12. Luftdekontaminationsvorrichtung (1) nach einem der Ansprüche 1 bis 11, **dadurch gekennzeichnet, dass** die Vorrichtung (1) ergänzende Kühlmittel umfasst, die dazu bestimmt sind, die Luft am Ausgang des Kompressors (4) ergänzend zum Kondensator (5) mit Zwangskonvektion abzukühlen.

13. Luftdekontaminationsvorrichtung (1) nach einem der Ansprüche 1 bis 12, **dadurch gekennzeichnet, dass** sie ein Bypass-Ventil (8) umfasst.

14. Luftdekontaminationsvorrichtung (1) nach einem der Ansprüche 1 bis 12, **dadurch gekennzeichnet, dass** sie einen Katalysator (3) umfasst, der stromaufwärts des Kompressors (4) platziert ist.

## Claims

1. A mobile airborne decontamination device (1) comprising:
(i) an inlet orifice and an outlet orifice,
(ii) dehumidification means, downstream of the inlet orifice, allowing dehumidifying the air entering the device (1) through said inlet orifice,
(iii) means for evaporating a microbicidal product, upstream of the outlet orifice and downstream of the dehumidification means, allowing vaporizing the microbicidal product in the air circulating in the device (1),
**characterized in that** the dehumidification means comprise a constant flow rate displacement compressor (4) connected to the inlet orifice and intended to compress the air entering the device (1), the compressor (4) being adapted to compress the air entering the device (1) up to a predetermined pressure (P), a forced-convection condenser (5) disposed downstream of the compressor (4), the condenser (5) being adapted to cool the air at the outlet of the compressor (4) and obtain the condensation at a temperature in the order of 20°C from the excess water contained in the previously compressed air, a water-separating filter (6) disposed downstream of the forced-convection condenser (5) for collecting the condensed water, the device (1) comprising adjusting means adapted to drop the pressure of the filtered air by the filter (6) up to a pressure lower than the predetermined pressure (P)

2. The airborne decontamination device (1) according to claim 1, **characterized in that** it comprises means for injecting the microbicidal product inside a duct (12a) of the evaporation means, the injection means comprising an injection needle (19) having a first end opening into the duct (12a) and a second end communicating with a hollow body (17a) intended to contain the microbicidal product, said injection means further comprising a piston (17b) capable of sliding inside the hollow body (17a) and a screw-nut system (26) actuated by a motor (20) for driving the translation of the piston (17b) in the hollow body (17a).

3. The airborne decontamination device (1) according to claim 2, **characterized in that** the device comprises a distribution valve (18) including three ways connected by a first way to the second end of the injection needle (19), by a second way to an ejection hole orifice (17c) located at the highest point of the hollow body (17a), and by a third way to a container (15).

4. The airborne decontamination device (1) according to claim 2 or 3, **characterized in that** the device (1) comprises means for indicating the position of the piston (17b).

5. The airborne decontamination device (1) according to claim 2, **characterized in that** the hollow body (17a) has, at the lowest point, a filling and draining orifice connected to a reversible pump (16) allowing filling or draining the hollow body (17a) with microbicidal product from the container (15).

6. The airborne decontamination device (1) according to any of claims 1 to 5, **characterized in that** the evaporation means comprise an evaporator (12) comprising the duct (12a) opening onto an upstream orifice (12b) and a downstream orifice (12c), said evaporation means further comprising heating means allowing heating the evaporator (12), the evaporator (12) corresponding to a metal block and the duct (12a) being machined in the metal block forming the evaporator (12).

7. The airborne decontamination device (1) according to claim 6, **characterized in that** the heating means comprise an electrical resistor (13) inserted in a bore (12e) of the metal block forming the evaporator (12).

8. The airborne decontamination device (1) according to any of claims 2 to 7, **characterized in that** the device (1) comprises an air heater (9) arranged in the duct (12a), upstream of an injection point (12d) where the injection needle (19) opens, in order to increase the temperature of the air before the injection of the microbicidal product.

9. The airborne decontamination device (1) according to any of claims 1 to 8, **characterized in that** the means for adjusting the discharge pressure comprise a valve (7).

10. The airborne decontamination device (1) according to any of claims 1 to 9, **characterized in that** the predetermined pressure (P) is greater than or equal to 3 bars (300kPa).

11. The airborne decontamination device (1) according to any of claims 1 to 10, **characterized in that** the forced convection condenser (5) comprises a metal tube (5a) and a fan (5b) allowing cooling the metal tube (5a) by propelling ambient air towards said metal tube (5a).

12. The airborne decontamination device (1) according to any of claims 1 to 11, **characterized in that** the device (1) of the complementary cooling means intended to cool the air at the outlet of the compressor (4) in addition to the forced-convection condenser (5).

13. The airborne decontamination device (1) according to any of claims 1 to 12, **characterized in that** it comprises a bypass valve (8).

14. The airborne decontamination device (1) according to any of claims 1 to 12, **characterized in that** it includes a catalyst (3) placed upstream of the compressor (4).
